# EUROPÄISCHE PATENTSCHRIFT

(11) **EP 1 634 554 B1**
(45) Veröffentlichungstag und Bekanntmachung des Hinweises auf die Patenterteilung: **15.04.2009**
(21) Anmeldenummer: 05018470.4
(22) Anmeldetag: 25.08.2005
(51) Int. Cl.: A61F 5/44, A61M 25/00

(54) **Urinbeutel**
Urine bag
Sac d'urine

(30) Priorität: 06.09.2004 DE 102004043434
(43) Veröffentlichungstag der Anmeldung: 15.03.2006
(73) Patentinhaber: Klaus Schmitt Beteiligungsgesellschaft mbH, 66557 Illingen (DE)
(72) Erfinder: Schmitt, Sandra Dr, 71065 Sindelfingen (DE)
(74) Vertreter: Vièl, Christof

(56) Entgegenhaltungen:
- WO-A-00/16843
- WO-A-01/43807
- WO-A-98/11932
- WO-A-03/096941
- DE-A1- 2 317 839
- DE-B- 2 022 107
- US-A- 2 856 932
- US-A- 4 515 593
- US-A- 4 723 944

## Beschreibung

Die Erfindung betrifft einen Urinbeutel, der einen Fortsatz für die sterile und trockene Aufbewahrung eines hydrophil beschichteten Katheters aufweist, wobei der Fortsatz an seinem freien Ende und im Übergangsbereich zu dem Urinbeutel Schließmittel aufweist.

Personen mit Querschnittslähmung sind nicht mehr in der Lage ihre Schließmuskel im urethravesikalen Bereich zu beeinflussen. Damit es nicht zum Hamstau und somit zu einer Schädigung der Niere oder einer Urosepsis kommt, muß die Harnblase durch Hilfsmittel entleert werden.

Dies kann mit Dauerkathetern geschehen, die ständig in der Harnröhre liegen und in der Harnblase mittels geblocktem Ballon arretiert sind und die im Rhythmus der natürlichen Miktion geöffnet werden um die Hamblase zu entleeren und anschließend wieder füllen zu lassen.

Durch die ständige Belastung der Harnröhre und die ausbleibende Spülung der urethralen Mukosa können Keime, in aller Regel Bakterien zwischen Schleimhaut und Katheter aufsteigen und so zu Infektionen führen.

Aus diesem Grund hat sich für diesen Patientenkreis die intermittierende Katheterisierung durchgesetzt. Bei dieser Technik wir 6 - 10 mal täglich ein sogenannter Nelaton-Katheter bis in die Harnblase geschoben und nach Entleerung wieder entfernt. Bei Einhaltung bestimmter steriler Kautelen können Infektionen weitgehend vermieden werden.

Stand der Technik ist, nach Reinigung des 0rificiums einen Nelaton-Katheter mit Gleitmittel zu versehen und dann den Katheter durch die Harnröhre bis in die Blase vorzuschieben um die Schließmuskel zu überwinden und die Blase zu entleeren. Der Urin wird dabei in sanitäre Einrichtungen geleitet oder in einen Urinbeutel aus Plastik, der mit dem Hausmüll entsorgt wird.

Um Infektionen vorzubeugen, gibt es Gleitmittel für den Katheter, die Chlorhexidin enthalten. Chlorhexidin ist bei einigen Fachleuten in Mißkredit geraten, da es im Verdacht steht kanzerogene Eigenschaften zu haben.

Eine Alternative hierzu sind hydrophil bcschichtete Katheter. Bei diesen Kathetern ist die Oberfläche mit einem getrockneten Gleitmittel überzogen, welches durch kurzes Einwirken von Wasser aktiviert wird und so den Katheter gleitfähig macht. Bei diesen Systemen muß steriles Wasser mitgeführt werden oder es wird Leitungswasser verwendet, bei dem jedoch die Gefahr der Verkeimung nicht auszuschließen ist.

Die Handhabung mit separat verpacktem sterilen Wasser ist in dem üblichen Umfeld von Toiletten jedoch äußerst kompliziert und es kommt oft trotz aller Vorsichtsmaßnamen zu Kontaminationen.

Mittlerweile sind auch Katheterisierungssets bekannt, bei denen der Katheter in einem Urinauffangbeutel untergebracht ist, der gleichzeitig mit einem Reservoir mit sterilem Wasser versehen ist. Diese Systeme sind schwierig zu handhaben und für Patienten, die ohnehin körperlich behindert sind, überhaupt nicht zu gebrauchen.

Aus der WO 98/11932 A1 ist ein Urinbeutel bekannt, der einen Fortsatz aufweist, in dem ein steriler und trockener hydrophil beschichteter Katheter aufbewahrt wird. Im Bereich des freien Endes des Fortsatzes sind im Übergangsbereich zum eigentlichen Urinbeutel Schließmittel vorgesehen. Zur Aktivierung des Katheters wird vor der Anwendung in den Schließmitteln eine Verbindung hergestellt, durch die vor dem Applizieren des Katheters eine in einem separat vom Urinbeutel ausgebildeten Volumen befindliche Flüssigkeit in den Fortsatz eingelassen wird, um die hydrophile Gleitschicht des Katheters zu aktivieren.

Die DE 23 17 839 A1 beschreibt einen hydrophil beschichteten Katheter, der sich in einem Fortsatz eines Beutels befindet. Aus einem von dem Urinbeutel getrennten Volumen wird vor dem Applizieren des Katheters über ein Ventil zur Aktivierung der hydrophilen Gleitschicht Flüssigkeit eingebracht.

Aus der WO 01/43807 A1 ist eine Benetzungsvorrichtung für einen Katheter bekannt, der einen Behälter für die Benetzungsflüssigkeit aufweist, welcher durch Zugkraft geöffnet werden kann und der in einem Benetzungsbehälter einen Harnröhrenkatheter enthält, welcher durch die Benetzungsflüssigkeit benetzt werden kann. Bei einer Ausführungsvariante ist zwischen dem Benetzungsbehälter und dem Harnröhrenkatheter ein Urinbeutel angeordnet.

Der Erfindung liegt die Aufgabe zugrunde, einen Urinbeutel zu schaffen, der einfach in der Anwendung ist und gleichzeitig alle hygienischen Anforderungen erfüllt. Darüber hinaus soll der Urinbeutel kostengünstig sein, um der Kostenreduzierung im Gesundheitswesen Rechnung zu tragen.

Diese Aufgabe wird erfindungsgemäß dadurch gelöst, daß zur Aktivierung vor der Anwendung die Schließmittel im Übergangsbereich zwischen dem Urinbeutel und dem Fortsatz zum Eintritt der in dem Urinbeutel befindlichen Flüssigkeit zum Aktivieren der hydrophilen Gleitschicht des Katheters in den Fortsatz öffenbar sind.

Es wird also ein Urinbeutel mit einer speziellen Form verwendet, dessen Reservoir in einen schmalen länglichen Fortsatz übergeht, welcher den Katheter mit hydrophiler Beschichtung aufnimmt. Der Fortsatz ist von dem Reservoir durch ein leicht zu öffnendes Schließmittel abgedichtet. Das Reservoir wird (beispielsweise über ein in der Schweißnaht des Reservoirs eingelassenes Ventil) mit soviel Aktivierungsflüssigkeit, z.B. Wasser oder einer wäßrigen Lösung, befüllt, daß der Fortsatz total geflutet werden kann und so das getrocknete hydrophile Gleitmittel aktiviert wird. Danach wird die Aktivierungsflüssigkeit wieder in das Reservoir zurückgeleitet. Nach Öffnen eines zweiten Schließmittels an dem freien Ende des Fortsatzes, kann nun der Katheter unter sterilen Kautelen seiner Zweckbestimmung zugeführt werden. Fortsatz und Reservoir dienen hierbei zur Aufnahme des Urins. Nach Entleerung der Blase wird der Katheter aus der Harnröhre entfernt und durch den Fortsatz in das Reservoir zurückgeschoben. Der Fortsatz kann durch einen Knoten verschlossen und das System hygienisch entsorgt werden. Der gesamte Urinbeutel mit dem in dem Fortsatz enthaltenen Katheter wird vorzugsweise in einem Peelbeutel sterilisiert und kommt dann steril verpackt zur Anwendung.

Hierbei liegt es im Rahmen der Erfindung, daß als Schließmittel eine Aufschiebeklammer vorgesehen ist, in die der Fortsatz eingefaltet oder eingerollt ist.

Derartige Aufschiebeklammern sind kostengünstig, dichten ausreichend ab und können leicht entfernt werden, um den Flüssigkeitsdurchgang zu ermöglichen.

Ebenso ist es möglich, daß der Fortsatz so in die Aufschiebeklammer eingerollt wird, daß bei Druckbelastung der mit Flüssigkeit befüllten Seite das System mit steigendem Druck immer fester schließt.

Zweckmäßig ist weiterhin, daß an dem dem Fortsatz gegenüberliegenden Ende des Urinbeutels ein Rückschlagventil eingelassen ist, welches die Befüllung des Urinbeutels mit Flüssigkeit erlaubt.

Hierbei ist es vorteilhaft, daß das Rückschlagventil ein Bunsenventil ist.

Unter einem Bunsenventil wird ein Ventil verstanden, das durch einen geschlitzten Schlauch gebildet ist, der in einer Richtung flüssigkeitsdurchgängig ist und in der anderen Richtung nicht

Weiterhin liegt es im Rahmen der Erfindung, daß das Schließmittel im Übergangsbereich zwischen dem Urinbeutel und dem Fortsatz als Rückschlagventil ausgebildet ist, welches durch Durchschieben des im Fortsatz befindlichen Katheters geöffnet wird, so daß die Aktivierungsflüssigkeit in den Fortsatz gelangt.

Ebenso ist es möglich, daß das Schließmittel im Übergangsbereich zwischen dem Urinbeutel und dem Fortsatz als Stopfen ausgebildet ist, der von der Seite des Fortsatzes her in den Urinbeutel zurückstoßbar ist und somit die Aktivierung erlaubt.

Bei Druckerhöhung im Beutel schließt der Stopfen immer dichter, während er von dem Fortsatz her, z.B. durch Drücken mit dem Katheter, geöffnet werden kann.

Eine vorteilhafte Weiterbildung der Erfindung besteht darin, daß die Flüssigkeit ein mikrobiozides Agens enthält.

Hierbei kann vorgesehen sein, daß das mikrobiozide Agens Silberverbindungen enthält.

Schließlich ist es zur Erfindung gehörig, daß die Silberverbindungen in kolloidaler Form oder in Form von Nanopartikeln enthalten sind.

Um ein weiteres Maß an Sicherheit einzubringen, kann die hydrophile Beschichtung des Katheters mit einer mikrobizid wirkenden Substanz, beispielsweise kolloidalem Silber oder kolloidalen Silberverbindungen oder auch solchen, die im Nanobereich liegen, versehen sein, um das Wachstum von Keimen, die trotz hygienischer Anwendungen in die Harnröhre eindringen, zu reduzieren bzw. diese Keime abzutöten.

Nachfolgend werden Ausführungsbeispiele der Erfindung anhand von Zeichnungen beschrieben.

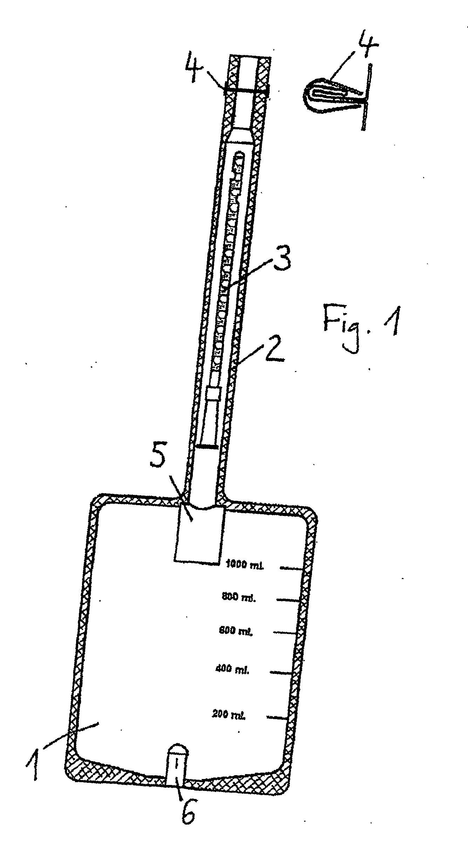

Es zeigen
- Fig. 1 bis Fig. 4: erfindungsgemäße Urinbeutel in geschnittener Darstellung mit verschiedenen Ausgestaltungen der Schließmittel.

Wie aus den Fig. 1 bis 4 ersichtlich, weist der erfindungsgemäße Urinbeutel 1 einen Fortsatz 2 für die sterile und trockene Aufbewahrung eines hydrophil beschichteten Katheters 3 auf, wobei der Fortsatz 2 an seinem freien Ende und im Übergangsbereich zu dem Urinbeutel Schließmittel 4, 5 aufweist. Zur Aktivierung vor der Anwendung ist im Übergangsbereich zwischen dem Urinbeutel 1 und dem Fortsatz 2 durch Öffnen des Schließmittels 5 eine Verbindung zwischen dem Urinbeutel 1 und dem Fortsatz 2 herstellbar. Durch diese tritt in dem Urinbeutel 1 befindliche Flüssigkeit zum Aktivieren der hydrophilen Gleitschicht des Katheters 3 in den Fortsatz 2 ein. Hierdurch wird der Katheter 3 erst kurz vor dem Gebrauch mit der Flüssigkeit benetzt, so daß die hydrophile Beschichtung des Katheters nicht durch zu langes Benetzen geschädigt wird.

Als Schließmittel 4, 5 sowohl für das freie Ende des Fortsatzes 2 als auch für den Übergangsbereich zwischen dem Urinbeutel 1 und dem Fortsatz 2 können beispielsweise Aufschiebeklammern vorgesehen sein, in die der Fortsatz eingefaltet ist. Diese Aufschiebeklammern sind U-förmig ausgebildet, wobei die freien Schenkel des Us aufeinander zulaufen. Zweckmäßigerweise wird der Fortsatz 2 so in die Aufschiebeklammer eingefaltet, daß bei Druckbelastung der mit Flüssigkeit befüllten Seite das System mit steigendem Druck immer fester schließt. Hierzu wird das freie Ende des Fortsatzes 2 vor dem Einführen in die Aufschiebeklammer zunächst um 180° umgeknickt, so daß der umgeknickte Endbereich auf dem mit Flüssigkeit gefüllten Bereich des Fortsatzes 2 liegt. Dann wird an der Knickstelle ein zweiter Knickvorgang vorgenommen, so daß das nunmehr geknickte Ende auf dem umgeknickten Endbereich zu liegen kommt. Anschließend wird der auf diese Weise geknickte Fortsatz 2 in die Aufschiebeklammer eingeführt.

Alternativ kann das freie Ende des Fortsatzes 2 auch aufgerollt werden und im aufgerollten Zustand in die Aufschiebeklammer eingeführt werden. Auch hierbei ist es vorteilhaft, wenn das freie Ende des Fortsatzes 2 zunächst um 180° umgeknickt wird und dann der Endbereich erst eingerollt wird, da sich hierdurch ebenfalls ein festeres Schließen bei Druckbelastung ergibt.

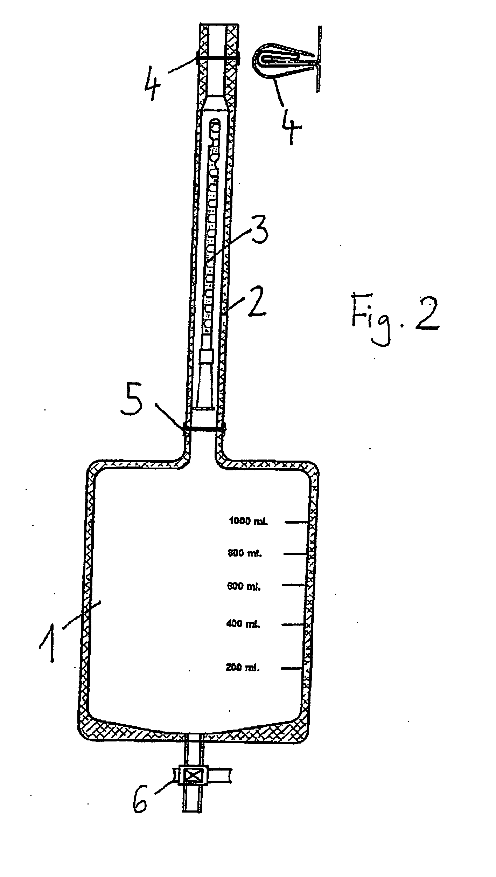

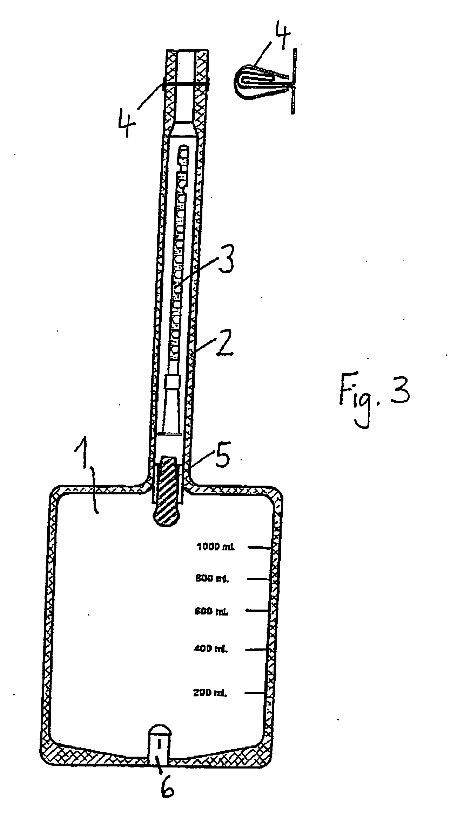

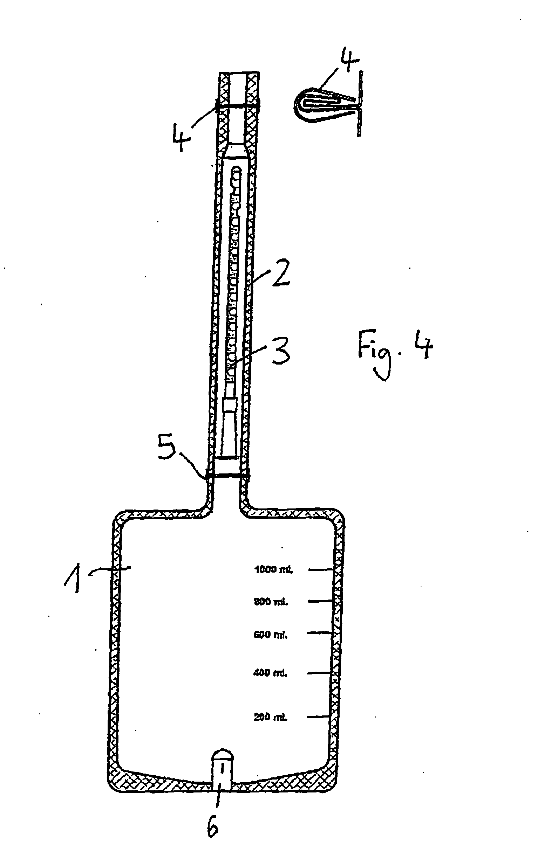

In Fig. 1 ist ein Urinbeutel 1 dargestellt, bei dem sowohl das freie Ende des Fortsatzes 2 als auch für den Übergangsbereich zwischen dem Urinbeutel 1 und dem Fortsatz 2 mit Aufschiebektammem 4, 5 als Schließmittel gesichert sind. In dem Übergangsbereich zwischen dem Urinbeutel 1 und dem Fortsatz 2 können alternativ zu einer Aufschiebeklammer 5 auch andere Schließmittel 5 angeordnet sein, z.B. als Rückschlagventil (Fig. 2 und 4), welches durch Durchschieben des im Fortsatz befindlichen Katheters geöffnet wird, so daß die Aktivierungsflüssigkeit in den Fortsatz 2 gelangen kann oder als Stopfen (Fig. 3), der von der Seite des Fortsatzes 2 her in den Urinbeutel 1 zurückgestoßen werden kann und somit die Aktivierung erlaubt.

An dem dem Fortsatz 2 gegenüberliegenden Ende des Urinbeutels 1 kann ein Rückschlagventil 6 eingelassen sein, welches die Befüllung des Urinbeutels 1 mit Flüssigkeit erlaubt und eine Rücklaufsperre besitzt. Dieses Rückschlagventil 6 kann beispielsweise als Bunsenventil 6 (Fig. 1, 3 und 4) ausgebildet sein, also als geschlitztes Schlauchsegment, welches nur in einer Richtung Flüssigkeit passieren läßt. Es kann auch als herkömmliches von Hand betätigbares Rückschlagventil 6 (Fig. 2) ausgebildet sein.

Vorzugsweise ist der Urinbeutel bereits mit der Flüssigkeit befüllt und die gesamte Einheit in einem Peelbeutel steril verpackt. Alternativ kann der Urinbeutel 1 auch erst vor der Anwendung über das Rückschlagventil 6 mit Flüssigkeit befüllt werden. In beiden Fällen ist es sinnvoll, daß die Flüssigkeit ein mikrobiozides Agens, beispielsweise Silberverbindungen, vorzugsweise in kolloidaler Form oder in Form von Nanopartikeln, enthält.

Darüber hinaus kann in dem Fortsatz ein Gleitgel eingebracht sein, das die Applikation des Katheters erleichtert.

## Patentansprüche

1. Urinbeutel, der einen Fortsatz (2) für die sterile und trockene Aufbewahrung eines hydrophil beschichteten Katheters (3) aufweist, wobei der Fortsatz (2) an seinem freien Ende und im Übergangsbereich zu dem Urinbeutel Schließmittel (4, 5) aufweist, **dadurch gekennzeichnet, daß** zur Aktivierung vor der Anwendung die Schließmittel (5) im Übergangsbereich zwischen dem Urinbeutel (1) und dem Fortsatz (2) zum Eintritt der in dem Urinbeutel (1) befindlichen Flüssigkeit zum Aktivieren der hydrophilen Gleitschicht des Katheters (3) in den Fortsatz (2) öffenbar sind.

2. Urinbeutel gemäß Anspruch 1, **dadurch gekennzeichnet, daß** als Schließmittel (4, 5) eine Aufschiebeklammer (4, 5) vorgesehen ist, in die der Fortsatz (2) eingefaltet oder eingerollt ist.

3. Urinbeutel gemäß Anspruch 2, **dadurch gekennzeichnet, daß** der Fortsatz (2) so in die Aufschiebeklammer (4, 5) eingefaltet wird, daß bei Druckbelastung der mit Flüssigkeit befüllten Seite das System mit steigendem Druck immer fester schließt.

4. Urinbeutel gemäß Anspruch 1, **dadurch gekennzeichnet, daß** an dem dem Fortsatz (2) gegenüberliegenden Ende des Urinbeutels (1) ein Rückschlagventil (6) eingelassen ist, welches die Befüllung des Urinbeutels (1) mit Flüssigkeit erlaubt.

5. Urinbeutel gemäß Anspruch 4, **dadurch gekennzeichnet, daß** das Rückschlagventil (6) ein Bunsenventil ist.

6. Urinbeutel gemäß Anspruch 1, **dadurch gekennzeichnet, daß** das Schließmittel (5) im Übergangsbereich zwischen dem Urinbeutel (1) und dem Fortsatz (2) als Rückschlagventil ausgebildet ist, welches durch Durchschieben des im Fortsatz (2) befindlichen Katheters (3) geöffnet wird, so daß die Aktivierungsflüssigkeit in den Fortsatz (2) gelangt.

7. Urinbeutel gemäß Anspruch 1, **dadurch gekennzeichnet, daß** das Schließmittel (5) im Übergangsbereich zwischen dem Urinbeutel (1) und dem Fortsatz (2) als Stopfen (5) ausgebildet ist, der von der Seite des Fortsatzes (2) her in den Urinbeutel (1) zurückstoßbar ist und somit die Aktivierung erlaubt.

8. Urinbeutel gemäß Anspruch 1, **dadurch gekennzeichnet, daß** die Flüssigkeit ein mikrobiozides Agens enthält.

9. Urinbeutel gemäß Anspruch 8, **dadurch gekennzeichnet, daß** das mikrobiozide Agens Silberverbindungen enthält.

10. Urinbeutel gemäß Anspruch 9, **dadurch gekennzeichnet, daß** die Silberverbindungen in kolloidaler Form oder in Form von Nanopartikeln enthalten sind.

## Claims

1. Urine bag, which has an extension (2) in which a catheter (3) with a hydrophilic coating is housed under sterile, dry conditions, the extension (2) having closure means (4, 5) at its free end and at the transition to the urine bag, **characterised in that** for the purpose of activation prior to use, the closure means (5) in the transition area between the urine bag (1) and the extension (2) can be opened to admit the liquid contained in the urine bag (1) for activating the hydrophilic, low-friction coating of the catheter (3) in the extension (2).

2. Urine bag according to claim 1, **characterised in that** a slip-on clip (4, 5) is provided as closure means (4, 5), into which the extension (2) is inserted in folded or rolled-up form.

3. Urine bag according to claim 2, **characterised in that** the extension (2) is folded into the slip-on clip (4, 5) in such a way that when pressure is exerted from the side filled with liquid, the system closes more and more tightly with increasing pressure.

4. Urine bag according to claim 1**, characterised in that,** at the end of the urine bag (1) that is opposite the extension (2), a check valve (6) is provided which permits filling of the urine bag (1) with liquid.

5. Urine bag according to claim 4, **characterized in that** the check valve (6) is a Bunsen valve.

6. Urine bag according to claim 1, **characterised in that** the closure means (5) in the transition area between the urine bag (1) and the extension (2) is configured as a check valve that is opened by pushing through the catheter (3) located in the extension (2), thus admitting activation liquid into the extension (2).

7. Urine bag according to claim 1, **characterised in that** the closure means (5) in the transition area between the urine bag (1) and the extension (2) is configured as a stopper (5) that can be pushed from the extension side (2) back into the urine bag (1), thus permitting activation.

8. Urine bag according to claim 1, **characterised in that** the liquid contains a microbicidal agent.

9. Urine bag according to claim 8, **characterised in that** the microbicidal agent contains silver compounds.

10. Urine bag according to claim 9, **characterised in that** the silver compounds are contained in colloidal form or in the form of nanoparticles.

## Revendications

1. Poche d'urine comportant un prolongement (2) pour la conservation stérile et à sec d'un cathéter (3) muni d'un revêtement hydrophile, le prolongement (2) étant muni de moyens de fermeture (4, 5) au niveau de son extrémité libre et dans la zone de transition avec la poche d'urine, **caractérisée en ce que,** pour l'activation avant l'utilisation, les moyens de fermeture (5), disposés dans la zone de transition entre la poche d'urine (1) et le prolongement (2), peuvent être ouverts pour que le liquide contenu dans la poche d'urine (1) pénètre dans le prolongement (2) pour activer la couche de glissement hydrophile du cathéter (3).

2. Poche d'urine selon la revendication 1, **caractérisée en ce qu'**il est prévu de réaliser les moyens de fermeture (4, 5) sous la forme d'une pince à enfiler (4, 5), dans laquelle le prolongement (2) est plié ou enroulé.

3. Poche d'urine selon la revendication 2, **caractérisée en ce que** le prolongement (2) est plié de telle sorte dans la pince à enfiler (4, 5) qu'en présence d'une contrainte en compression du côté rempli de liquide, le système se ferme de plus en plus fermement sous l'effet de l'augmentation de la pression.

4. Poche d'urine selon la revendication 1, **caractérisée en ce qu'**un clapet de non-retour (6) est inséré au niveau de l'extrémité de la poche d'urine (1) opposée au prolongement (2), lequel clapet permet de remplir la poche d'urine (1) avec un liquide.

5. Poche d'urine selon la revendication 4, **caractérisée en ce que** le clapet de non-retour (6) est un clapet de Bunsen.

6. Poche d'urine selon la revendication 1, **caractérisée en ce que** les moyens de fermeture (5) dans la zone de transition entre la poche d'urine (1) et le prolongement (2) sont réalisés sous la forme d'un clapet de non-retour qui s'ouvre sous l'effet de la poussée à travers du cathéter (3) situé dans le prolongement (2), de telle sorte que le liquide d'activation pénètre dans le prolongement (2).

7. Poche d'urine selon la revendication 1, **caractérisée en ce que** les moyens de fermeture (5) dans la zone de transition entre la poche d'urine (1) et le prolongement (2) sont réalisés sous la forme d'un bouchon (5) qui peut être repoussé à l'intérieur de la poche d'urine (1) à partir du côté du prolongement (2) et permet ainsi l'activation.

8. Poche d'urine selon la revendication 1, **caractérisée en ce que** le liquide contient un agent microbiocide.

9. Poche d'urine selon la revendication 8, **caractérisée en ce que** l'agent microbiocide contient des composés d'argent.

10. Poche d'urine selon la revendication 9, **caractérisée en ce que** les composés d'argent sont contenus sous forme colloïdale ou sous forme de nanoparticules.
